(19) Europäisches Patentamt
European Patent Office
Office européen des brevets

(11) Veröffentlichungsnummer: **0 497 133 A1**

(12) **EUROPÄISCHE PATENTANMELDUNG**

(21) Anmeldenummer: **92100482.6**

(22) Anmeldetag: **14.01.92**

(51) Int. Cl.5: **C07C 211/28**, C07C 211/29, C07C 209/68

(30) Priorität: **26.01.91 DE 4102289**

(43) Veröffentlichungstag der Anmeldung: **05.08.92 Patentblatt 92/32**

(84) Benannte Vertragsstaaten: **BE CH DE ES FR GB IT LI NL**

(71) Anmelder: **BAYER AG**

**W-5090 Leverkusen 1 Bayerwerk(DE)**

(72) Erfinder: **Kuhnt, Dietmar, Dr.**
**Körnerstrasse 5**
**W-5090 Levekusen(DE)**
Erfinder: **Himmler, Thomas, Dr.**
**Schöne Aussicht 1b**
**W-5068 Odenthal-Glöbusch(DE)**
Erfinder: **Ziemann, Heinz, Dr.**
**Am Wiesenberg 10**
**W-5653 Leichlingen(DE)**

(54) **Verfahren zur Herstellung von (Hetero)Arylalk(en/in)-ylaminen und neue (Hetero)Arylalkinylamine.**

(57) Die Erfindung betrifft ein neues Verfahren zur Herstellung von (Hetero)Arylalk(en/in)ylaminen der Formel (I)

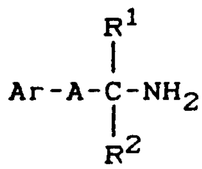

(I)

in welcher

A für Ethan-1,2-diyl (Ethylen, Dimethylen, $-CH_2-CH_2-$) oder für Ethen-1,2-diyl (Ethenylen, Vinylen, $-CH=CH-$) oder für Ethin-1,2-diyl (Ethinylen, $-C\equiv C-$) steht,

dadurch gekennzeichnet, daß man

(a) für den Fall, daß in der Formel (I) A für Ethin-1,2-diyl steht

Halogen(hetero)arylverbindungen der allgemeinen Formel (II)

Ar-X (II)

mit Aminoalkinylverbindungen der allgemeinen Formel (III)

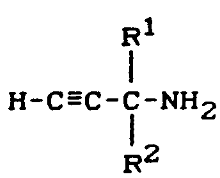

(III)

umsetzt

EP 0 497 133 A1

und gegebenenfalls

(b) für den Fall, daß in der Formel (I) A für Ethan-1,2-diyl oder Ethen-1,2-diyl steht

die nach dem unter (a) beschriebenen Verfahrens-schritt erhaltenen neuen (Hetero)Aralkinylamine der allgemeinen Formel (Ia)

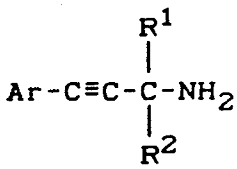

(Ia)

mit einem Hydrierungsmittel, gegebenenfalls in Gegenwart eines Katalysators und gegebenenfalls in Gegenwart eines Verdünnungsmittels umsetzt, wobei für Ar, $R^1$, $R^2$ und X die in der Beschreibung genannten Definitionen gelten, sowie deren Verwendung als Zwischenprodukte für Arzneimittel und Pflanzenschutzmittel, hier insbesonder Herbizide.

Die Erfindung betrifft ein neues Verfahren zur Herstellung von (Hetero)Arylalk(en/in)ylaminen sowie neue (Hetero)Arylalkinylamine, welche als Zwischenprodukte für Arzneimittel und Pflanzenschutzmittel, hier insbesondere Herbizide, verwendet werden können.

Es ist bekannt, daß man bestimmte Arylalkinylamine, wie z.B. N-Ethyl-1,1-dimethyl-3-phenyl-2-propinylamin, erhält, wenn man geeignete Halogenverbindungen, wie z.B. 1-Chlor-1,1-dimethyl-3-phenyl-2-propin, mit Aminoverbindungen, wie z.B. Ethylamin, umsetzt (vgl. J. Org. Chem. 31 (1966), 122-127, insbesondere S. 123). Die Ausbeute bei dieser Umsetzung ist jedoch sehr gering.

Weiter ist bekannt, daß man bestimmte Hydroxyalkinylarylverbindungen, wie z.B.

N-[3-Chlor-4-(3'-methyl-3'-hydroxybut-1'-in-1'-yl)-phenyll-N'-methoxy-N'-methyl-harnstoff, erhält, wenn man geeignete Halogenarylverbindungen, wie z.B. N-(3'-Chlor-4'-bromphenyl)-N'-methoxy-N'-methylharnstoff, mit Hydroxyalkinylverbindungen, wie z.B. 3-Hydroxy-3-methyl-1-butin, umsetzt (vgl. EP-A 30922, insbesondere S. 9). Eine analoge Umsetzung zur Herstellung von Aminoalkinylarylverbindungen ist jedoch bisher nicht bekannt.

Gegenstand der vorliegenden Anmeldung ist ein neues Verfahren zur Herstellung von (Hetero)Arylalk-(en/in)ylaminen der allgemeinen Formel (I)

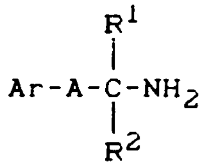

(I)

in welcher

A für Ethan-1,2-diyl (Ethylen, Dimethylen, $-CH_2-CH_2-$) oder für Ethen-1,2-diyl (Ethenylen, Vinylen, -CH = CH-) oder für Ethin-1,2-diyl (Ethinylen, -C≡C-) steht,

Ar für jeweils gegebenenfalls substituiertes Aryl oder Heteroaryl steht, sowie

$R^1$ und $R^2$ gleich oder verschieden sind und einzeln für Wasserstoff oder Alkyl stehen oder zusammen für Alkandiyl (Alkylen) stehen,

dadurch gekennzeichnet, daß man

(a) für den Fall, daß in der Formel (I) A für Ethin-1,2-diyl steht und Ar, $R^1$ und $R^2$ die oben angegebene Bedeutung haben,

Halogen(hetero)arylverbindungen der allgemeinen Formel (II)

Ar-X (II)

in welcher

Ar die oben angegebene Bedeutung hat und

X für Halogen steht,

mit Aminoalkinylverbindungen der allgemeinen Formel (III)

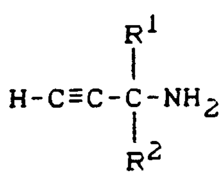

(III)

in welcher

$R^1$ und $R^2$ die oben angegebene Bedeutung haben,

in Gegenwart eines Katalysators, in Gegenwart eines Säureakzeptors, gegebenenfalls in Gegenwart weiterer Reaktionshilfsmittel und gegebenenfalls in Gegenwart eines Verdünnungsmittels bei Temperaturen zwischen 0 ° C und 200 ° C umsetzt,

und gegebenenfalls

(b) für den Fall, daß in der Formel (I) A für Ethan-1,2-diyl oder Ethen-1,2-diyl steht und Ar, $R^1$ und $R^2$ die oben angegebene Bedeutung haben,

die nach dem unter (a) beschriebenen Verfahrens-schritt erhaltenen Verbindungen der allgemeinen Formel (Ia)

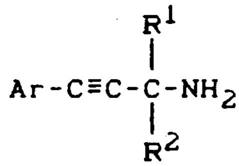

(Ia)

in welcher

Ar, $R^1$ und $R^2$ die oben angegebene Bedeutung haben,

mit einem Hydrierungsmittel, gegebenenfalls in Gegenwart eines Katalysators und gegebenenfalls in Gegenwart eines Verdünnungsmittels, bei Temperaturen zwischen 0 ° C und 150 ° C umsetzt.

Überraschenderweise können nach dem erfindungsgemäßen Verfahren im Verfahrensschritt (a) die (Hetero)Arylalkinylamine der Formel (Ia) auf einfache Weise in hohen Ausbeuten und in sehr guter Qualität hergestellt werden. Der als Nebenreaktion zu erwartende nucleophile Angriff der Aminogruppe der Verbindungen der Formel (III) an der halogenierten Position der Halogen(hetero)arylverbindungen der Formel (II) wird nicht in nennenswertem Umfang beobachtet.

Das erfindungsgemäße Verfahren stellt damit - sowohl als Verfahrensschritt (a) allein als auch in der Kombination der Verfahrensschritte (a) und (b) - eine wertvolle Bereicherung des Standes der Technik dar.

Die nach dem oben unter (a) beschriebenen Verfahrensschritt herzustellenden Verbindungen der Formel (Ia)

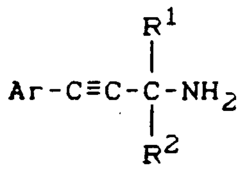

(Ia)

in welcher

Ar für jeweils gegebenenfalls substituiertes Aryl oder Heteroaryl steht und

$R^1$ und $R^2$ gleich oder verschieden sind und einzeln für Wasserstoff oder Alkyl stehen oder zusammen für Alkandiyl (Alkylen) stehen,

sind mit Ausnahme von 3-Phenyl-2-propinylamin und 1-Methyl-3-phenyl-2-propinylamin (beide bekannt aus J. Med. Chem. 13 (1970), 1249-1250) sowie N'-[3-(3-Amino-3-methyl-1-butinyl)-phenyl]-N-methoxy-N-methyl-harnstoff (bekannt aus EP-A 30922, Beispiel 2.70, S. 19) neu und als neue Stoffe Gegenstand der vorliegenden Anmeldung.

Diese neuen (Hetero)Arylalkinylamine der Formel (Ia) können als Zwischenprodukte zur Herstellung von Arzneimitteln und Pflanzenschutzmitteln verwendet werden.

Nach dem erfindungsgemäßen Verfahren werden vorzugsweise Verbindungen der Formel (I) hergestellt, in welcher

A für Ethan-1,2-diyl (Ethylen, Dimethylen, $-CH_2-CH_2-$) oder für Ethen-1,2-diyl (Ethenylen, Vinylen, $-CH=CH-$) oder für Ethin-1,2-diyl (Ethinylen, $-C\equiv C-$) steht,

Ar für jeweils gegebenenfalls substituiertes Aryl oder Heteroaryl mit jeweils bis zu 10 Kohlenstoffatomen und gegebenenfalls 1 bis 3 Stickstoffatomen und/oder einem Sauerstoff- oder Schwefelatom als Heteroatom(en) steht, wobei als bevorzugte Substituenten ausgewählt sind:

Halogen, Cyano, Nitro, jeweils geradkettiges oder verzweigtes Alkyl, Alkoxy, Alkylthio oder Dialkylamino mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 8 Kohlenstoffatomen in den einzelnen Alkylteilen, Cycloalkyl mit 3 bis 7 Kohlenstoffatomen, zweifach verknüpftes Alkandiyl mit 3 bis 5 Kohlenstoffatomen, gegebenenfalls einfach bis vierfach durch Fluor und/oder Chlor substituiertes Alkylendioxy mit 1 oder 2 Kohlenstoffatomen, jeweils gegebenenfalls im Arylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy, Aryloxyalkyl oder Aralkyloxy mit jeweils 6

bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Heteroarylalkyl oder Heteroaryl mit jeweils 2 bis 9 Kohlenstoffatomen und 1 bis 4 gleichen oder verschiedenen Heteroatomen - insbesondere Stickstoff, Sauerstoff und/oder Schwefel - im Heteroarylteil und gegebenenfalls 1 bis 4 Kohlenstoffatomen im geradkettigen oder verzweigten Alkylteil; sowie

$R^1$ und $R^2$ gleich oder verschieden sind und einzeln für Wasserstoff oder für geradkettiges oder verzweigtes Alkyl mit 1 bis 6 Kohlenstoffatomen stehen oder zusammen für Alkandiyl (Alkylen) mit 2 bis 6 Kohlenstoffatomen stehen.

Insbesondere werden nach dem erfindungsgemäßen Verfahren Verbindungen der Formel (I) hergestellt, in welcher

A für Ethan-1,2-diyl (Ethylen, Dimethylen, $-CH_2-CH_2-$) oder für Ethen-1,2-diyl (Ethenylen, Vinylen, $-CH=CH-$) oder für Ethin-1,2-diyl (Ethinylen, $-C\equiv C-$) steht,

Ar für jeweils gegebenenfalls einfach bis dreifach substituiertes Phenyl, Naphthyl, Pyridyl, Pyrimidyl, Triazinyl, Furyl, (Iso)Oxazolyl, Oxadiazolyl, Thienyl, (Iso)Thiazolyl oder Thiadiazolyl steht, wobei als besonders bevorzugte Substituenten ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Dimethylamino, Difluormethyl, Trifluormethyl, Difluormethoxy, Trifluormethoxy, Difluormethylthio, Trifluormethylthio, Methoxycarbonyl, Ethoxycarbonyl, n- oder i-Propoxycarbonyl, Methoximinomethyl, Ethoximinomethyl, Methoximinoethyl, Ethoximinoethyl, Cyclohexyl oder jeweils gegebenenfalls im Phenylteil ein- bis dreifach, gleich oder verschieden durch Fluor, Chlor, Brom, Methyl, Ethyl, Methoxy, Ethoxy, Methylthio, Trifluormethyl, Difluormethoxy, Trifluormethoxy und/oder Trifluormethylthio substituiertes Phenyl, Benzyl, Phenoxy, Phenoxymethyl oder Benzyloxy,

$R^1$ für Wasserstoff, Methyl, Ethyl, n- oder i-Propyl steht und

$R^2$ für Methyl, Ethyl, n- oder i-Propyl, n- oder i-Butyl steht, oder zusammen mit $R^1$ für Ethan-1,2-diyl (Dimethylen), Propan-1,3-diyl (Trimethylen), Butan-1,4-diyl (Tetramethylen) oder Pentan-1,5-diyl (Pentamethylen) steht.

Als neue Stoffe der Formel (Ia) sind bevorzugt bzw. besonders bevorzugt diejenigen Verbindungen der Formel (Ia), in welcher Ar, $R^1$ und $R^2$ diejenigen Bedeutungen haben, die oben im Zusammenhang mit der Beschreibung der erfindungsgemäß herzustellenden Verbindungen der Formel (I) vorzugsweise bzw. als insbesondere bevorzugt für Ar, $R^1$ und $R^2$ angegeben sind, wobei die bekannten Verbindungen 3-Phenyl-2-propinylamin und 1-Methyl-3-phenyl-2-propinylamin durch Disclaimer ausgenommen sind.

Verwendet man in der oben unter (a) beschriebenen Stufe des erfindungsgemäßen Verfahrens beispielsweise Brombenzol und 3-Amino-3-methyl-1-propin als Ausgangsstoffe und anschließend gemäß der oben unter (b) beschriebenen Verfahrensstufe ein bzw. zwei Moläquivalent(e) Wasserstoff als Hydrierungsmittel, so kann der Reaktionsablauf beim erfindungsgemäßen Verfahren durch das folgende Formelschema skizziert werden:

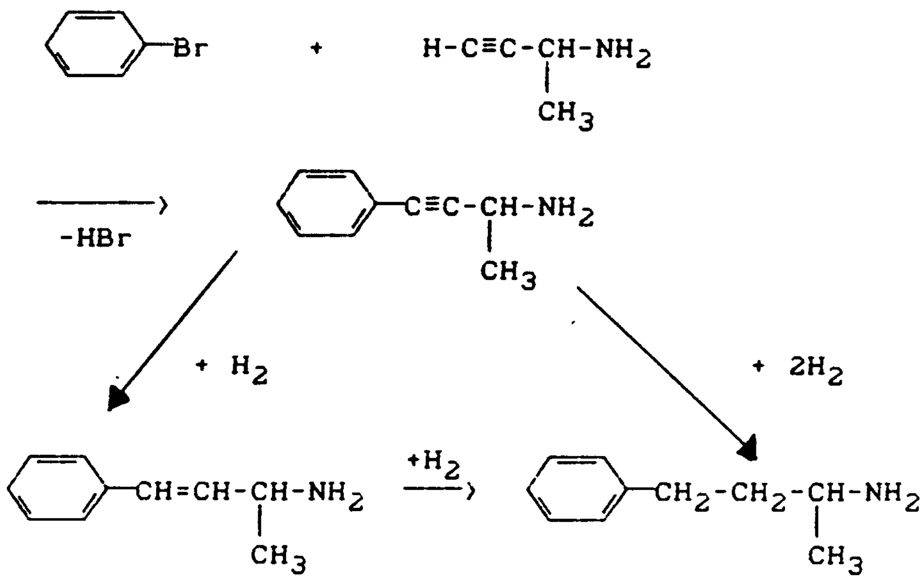


In der Folge wird die oben unter (a) beschriebene Verfahrensstufe als Verfahren (a), die unter (b) beschriebene Stufe als Verfahren (b) bezeichnet.

Die beim erfindungsgemäßen Verfahren (a) zur Herstellung von Verbindungen der Formel (I) als Ausgangsstoffe zu verwendenden Halogen(hetero)arylverbindungen sind durch die Formel (II) allgemein definiert.

In Formel (II) hat Ar vorzugsweise bzw. insbesondere diejenige Bedeutung, die bereits oben im Zusammenhang mit der Beschreibung der erfindungsgemäß herzustellenden Verbindungen der Formel (I) vorzugsweise bzw. als insbesondere bevorzugt für Ar angegeben wurde; X steht vorzugsweise für Chlor, Brom oder Jod, insbesondere für Brom oder jod.

Als Beispiele für die Ausgangsstoffe der Formel (II) seien genannt:
Chlor-, Brom- und Jod-benzol, 3-Fluor-, 4-Fluor- und 3,4-Difluor-brombenzol, 3-Chlor- und 4-Chlor-brombenzol, 3-Brom- und 4-Brom-benzonitril, 3-Brom- und 4-Brom-toluol, 3-Brom- und 4-Brom-1-methoxy-benzol, 3-Brom- und 4-Brom-benzotrifluorid, 4-Brom-benzoesäure-methylester, 2-Brom-, 3-Brom- und 4-Brom-pyridin, 1-Brom- und 2-Brom-naphthalin, 2-Brom-, 4-Brom- und 5-Brom-pyrimidin, 2-Brom- und 3-Brom-furan, 2-Brom-, 4-Brom- und 5-Brom-oxazol, 2-Brom- und 3-Brom-thiophen, 2-Chlor-4,6-dimethyl-pyrimidin.

Die Ausgangsstoffe der Formel (II) sind bekannte organische Synthesechemikalien.

Die beim erfindungsgemäßen Verfahren (a) zur Herstellung von Verbindungen der Formel (I) weiter als Ausgangsstoffe zu verwendenden Aminoalkinylverbindungen sind durch die Formel (III) allgemein definiert.

In Formel (III) haben $R^1$ und $R^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$ und $R^2$ angegeben wurden.

Als Beispiele für die Ausgangsstoffe der Formel (III) seien genannt:
3-Amino-3-methyl-1-propin, 3-Amino-3-ethyl-1-propin, 3-Amino-3,3-dimethyl-1-propin, 3-Amino-3,3-diethyl-1-propin, 3-Amino-3-methyl-3-ethyl-1-propin, 3-Amino-3-methyl-3-propyl-1-propin und 1-Amino-1-ethinyl-cyclohexan.

Die Ausgangsstoffe der Formel (III) sind bekannte organische Synthesechemikalien.

Das erfindungsgemäße Verfahren (a) wird in Gegenwart eines Katalysators durchgeführt. Als Katalysatoren kommen hierbei vorzugsweise Edelmetalle, wie z.B. Silber, Gold, Ruthenium, Rhodium, Palladium, Osmium, Iridium und Platin, insbesondere Palladium und Platin, aber auch Edelmetallverbindungen, wie z.B. Palladium(II)-acetat, Palladium(II)-acetylacetonat, Palladium(II)-bromid, Palladium(II)-chlorid, Palladium(II)-iodid, Palladium(II)-oxid, Palladium(II)-sulfat, Palladium(O)-tetrakis(triphenylphosphin), Palladium(II)-bis-(triphenylphosphin)-dichlorid, Platin(II)-acetylacetonat, Platin(II)-bis-(triethylphosphin)-dichlorid, Platin(II)-bis-(triphenylphosphin)-dichlorid, Platin(II)-bromid, Platin(II)-chlorid, Platin(II)-iodid, Platin(IV)-oxid und Platin(O)-tetrakis(triphenylphosphin), in Betracht.

Als Säureakzeptoren können bei dem erfindungsgemäßen Verfahren (a) alle üblicherweise für derartige Umsetzungen verwendbaren Säurebindemittel eingesetzt werden. Vorzugsweise infrage kommen Alkalimetall- und Erdalkalimetallhydride, wie Lithium-, Natrium-, Kalium- und Calcium-hydrid, Alkalimetall- und Erdalkalimetall-hydroxide, wie Lithium-, Natrium-, Kalium- und Calcium-hydroxid, Alkalimetall- und

Erdalkalimetall-carbonate und -hydrogencarbonate, wie Natrium- und Kalium-carbonat oder -hydrogencarbonat sowie Calciumcarbonat, Alkalimetall-acetate, wie Natrium- und Kalium-acetat, Alkalimetall-alkoholate, wie Natrium- und Kalium-methylat, -ethylat, -propylat, -isopropylat, -butylat, -isobutylat und tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl- 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Das erfindungsgemäße Verfahren (a) wird gegebenenfalls in Gegenwart weiterer Reaktionshilfsmittel durchgeführt. Als solche kommen vor allem Kupfer und/oder Kupferverbindungen, wie z.B. Kupfer(I)-chlorid, Kupfer(I)-bromid, Kupfer(I)-iodid und Kupfer(I)-oxid, und gegebenenfalls auch zusätzlich Trialkyl- oder Triarylphosphine, wie z.B. Tributylphosphin oder Triphenylphosphin, aber auch Phasentransfer-Katalysatoren, wie z.B. Benzyltributylammonium-chlorid und -bromid. Benzyltriethylammoniumchlorid, Butyltriphenylphosphoniumchlorid, Ethyltrioctylphosphoniumbromid, Hexadecyltrimethylammoniumbromid, Methyltrioctylammoniumchlorid und Tetrabutylammoniumbromid in Betracht.

Das erfindungsgemäße Verfahren (a) wird vorzugsweise in Gegenwart eines Verdünnungsmittels durchgeführt. Als Verdünnungsmittel kommen dabei praktisch alle inerten organischen Lösungsmittel, vorzugsweise jedoch aprotisch polare Solventien, wie z.B. Dibutylether, Glycoldimethylether, Diglycoldimethylether, Tetrahydrofuran, Dioxan, Essigsäureethylester, -propylester, -isopropylester, -butylester, -isobutylester und -sec-butylester, Acetonitril und Propionitril, Dimethylformamid, Dimethylacetamid, N-Methyl-pyrrolidon, Dimethylsulfoxid und Tetramethylensulfon in Betracht. Soweit basische Stickstoffverbindungen als Säureakzeptoren verwendet werden, können diese auch - im Überschuß eingesetzt - als Verdünnungsmittel dienen.

Die Reaktionstemperaturen können bei dem erfindungsgemäßen Verfahren (a) in einem größeren Bereich variiert werden. Im allgemeinen arbeitet man bei Temperaturen zwischen 0 °C und 200 °C, vorzugsweise bei Temperaturen zwischen 20 °C und 150 °C.

Das erfindungsgemäße Verfahren (a) wird im allgemeinen unter Normaldruck durchgeführt. Es ist jedoch auch möglich, unter erhöhtem oder vermindertem Druck - im allgemeinen zwischen 10 mbar und 10000 mbar - zu arbeiten.

Zur Durchführung des erfindungsgemäßen Verfahrens (a) setzt man auf 1 Mol Halogen(hetero)-arylverbindung der Formel (II) im allgemeinen zwischen 0,5 und 1,5 Mol, vorzugsweise zwischen 0,9 und 1,3 Mol, einer Aminoalkinylverbindung der Formel (III) sowie zwischen 0,001 und 0,1 Mol, vorzugsweise zwischen 0,005 und 0,05 Mol, eines Katalysators ein.

Die Reaktionskomponenten können zur Durchführung von Verfahren (a) in beliebiger Reihenfolge zusammengegeben werden.

In einer bevorzugten Ausführungsform des erfindungsgemäßen Verfahrens (a) werden die Ausgangsstoffe der Formeln (II) und (III) bei Temperaturen zwischen 0 °C und 50 °C, vorzugsweise zwischen 10 °C und 30 °C, mit dem Verdünnungsmittel, welches gegebenenfalls mit dem Säureakzeptor identisch ist, vermischt. Dann wird der Katalysator und gegebenenfalls ein oder mehrere weitere Reaktionshilfsmittel sowie gegebenenfalls ein Säureakzeptor dazu gegeben und das Reaktionsgemisch wird dann bei erhöhter Temperatur zwischen 50 °C und 200 °C, vorzugsweise zwischen 80 °C und 150 °C, bis zum Ende der Umsetzung gerührt. Die Aufarbeitung kann dann nach üblichen Methoden durchgeführt werden.

Beispielsweise wird filtriert, das Filtrat unter vermindertem Druck eingeengt, der Rückstand mit Wasser und einem mit Wasser praktisch nicht mischbaren organischen Lösungsmittel, wie z.B. Methylenchlorid, geschüttelt, die organische Phase abgetrennt, getrocknet und filtriert. Vom Filtrat wird das Lösungsmittel im Wasserstrahlvakuum sorgfältig abdestilliert. Das verbleibende Rohprodukt kann auf übliche Weise, z.B. durch Vakuumdestillation, weiter gereinigt werden.

Das erfindungsgemäße Verfahren (b) wird unter Verwendung eines Hydrierungsmittels durchgeführt. Es kommen hierbei die üblichen zur Hydrierung von Alkinen geeigneten Mittel in Betracht. Hierzu gehören z.B. Natrium in flüssigem Ammoniak, Natriumboranat, Natriumformiat, Cyclohexen, Hydrazin, Hydroxylamin und Wasserstoff. Vorzugsweise wird Verfahren (b) als katalytische Hydrierung mit Wasserstoff in Gegenwart eines geeigneten Katalysators durchgeführt.

Für Verfahren (b) geeignete Katalysatoren sind die üblicherweise bei katalytischen Hydrierungen verwendeten Metalle oder Metallverbindungen. Soweit die Hydrierung zu gesättigten Produkten (I, A = Ethan-1,2-diyl) führen soll, wird sie vorzugsweise unter Verwendung von Raney-Nickel, Palladium oder Platin, gegebenenfalls auf geeignetem Trägermaterial, wie z.B. Aktivkohle, durchgeführt. Falls die Hydrierung nur bis zur Alken-Stufe (I, A = Ethen-1,2-diyl) führen soll, werden vorzugsweise modifizierte Katalysatoren, wie z.B. der sogenannte LindlarKatalysator (Palladium auf Calciumcarbonat, gegebenenfalls mit einer Bleiverbindung, wie z.B. Blei(II)-acetat, dotiert) verwendet (zu Hydrierungsmitteln und Katalysatoren vgl. Houben-Weyl, Methoden der organischen Chemie, 4. Auflage, Band 5/2a (1977), S. 687-700, Georg-Thieme-Verlag, Stuttgart).

Verfahren (b) wird vorzugsweise in Gegenwart eines Verdünnungsmittels durchgeführt. Vorzugsweise werden Alkohole, wie Methanol, Ethanol, Propanol und Isopropanol, Ether, wie Glycoldimethylether, Diglycoldimethylether, Tetrahydrofuran und Dioxan, oder Ester, wie Essigsäuremethylester, -ethylester, -propylester, -isopropylester, -butylester, -isobutylester und -sec-butylester als Verdünnungsmittel für Verfahren (b) eingesetzt.

Die Reaktionstemperaturen können bei dem erfindungsgemäßen Verfahren (b) in einem größeren Bereich variiert werden. Im allgemeinen arbeitet man bei Temperaturen zwischen 0 °C und 150 °C, vorzugsweise bei Temperaturen zwischen 10 °C und 80 °C.

Verfahren (b) wird im allgemeinen bei Normaldruck oder erhöhtem Druck bis etwa 200 bar, vorzugsweise bis etwa 100 bar durchgeführt.

Verfahren (b) wird unter für Hydrierungen üblichen Reaktionsbedingungen durchgeführt. In einer bevorzugten Ausführungsform von Verfahren (b) wird das Zwischenprodukt der Formel (Ia) mit einem Katalysator und einem Verdünnungsmittel vermischt und in diese Mischung wird das Hydrierungsmittel unter Rühren eindosiert, bis ein bzw. zwei Moläquivalente davon umgesetzt sind.

Die Aufarbeitung kann auf übliche Weise durchgeführt werden. Beispielsweise wird filtriert, das Filtrat unter vermindertem Druck eingeengt und gegebenenfalls das verbleibende Rohprodukt durch Vakuumdestillation weiter gereinigt.

Die nach dem erfindungsgemäßen Verfahren herzustellenden (Hetero)Arylalk(en/in)ylamine der Formel (I) können als Zwischenprodukte für Arzneimittel und Pflanzenschutzmittel verwendet werden (vgl. DE-OS 3434271, EP-A 294666, Herstellungsbeispiele).

Herstellungsbeispiele:

Beispiel 1

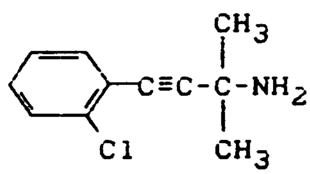

(Verfahren (a))

96,7 g (0,50 Mol) 1-Brom-2-chlor-benzol und 45,7 g (0,55 Mol) 3-Amino-3,3-dimethyl-1-propin werden in 500 ml Triethylamin vorgelegt. Nach Zugabe von 7,0 g (0,01 Mol) Palladium(II)-bis(triphenylphosphin)-dichlorid, 7,6 g (0,04 Mol) Kupfer(I)-iodid und 21,0 g (0,4 Mol) Triphenylphosphin wird das Reaktionsgemisch 24 Stunden unter Rückfluß erhitzt. Dann wird filtriert und das Filtrat im Wasserstrahlvakuum eingeengt. Der Rückstand wird mit Methylenchlorid/Wasser (ca. 300 ml/300 ml) extrahiert, die organische Phase abgetrennt, mit Natriumsulfat getrocknet und filtriert. Vom Filtrat wird das Lösungsmittel im Wasserstrahlvakuum abdestilliert und das verbleibende Rohprodukt durch Destillation im Ölpumpenvakuum gereinigt.

Man erhält 74,1 g (75% der Theorie) 3-Amino-3,3-dimethyl-1-(2-chlor-phenyl)-1-propin vom Brechungsindex $n_D^{21}$ = 1,5799, mit einem Siedebereich von Kp: 73-75 °C bei 0,1 mbar.

Beispiel 2

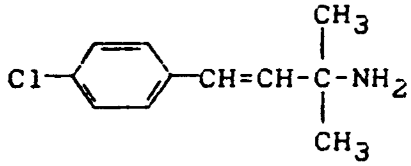

(Verfahren (b))

96,8 g (0,50 Mol) 3-Amino-3,3-dimethyl-1-(4-chlor-phenyl)-1-propin werden in einer Hydrierapparatur nach Parr mit 400 ml Tetrahydrofuran vermischt und mit 13,0 g Lindlar-Katalysator (5% Palladium auf Calciumcarbonat mit Blei dotiert) versetzt. Das Gemisch wird dann unter einem Wasserstoffdruck von 3 bar bei allmählich von 25 °C auf 50 °C erhöhter Temperatur bis zum Ende der berechneten Wasserstoff-Aufnahme (1 Moläquivalent nach etwa 15 Stunden) geschüttelt. Dann wird filtriert, das Filtrat im Wasserstrahlvakuum eingeengt und das verbleibende Rohprodukt durch Destillation im Ölpumpenvakuum gereinigt.

Man erhält 67,5 g (69% der Theorie) 3-Amino-3,3-dimethyl-1-(4-chlor-phenyl)-1-propen vom Brechungsindex $n_D^{21}$ = 1,5528.

Beispiel 3

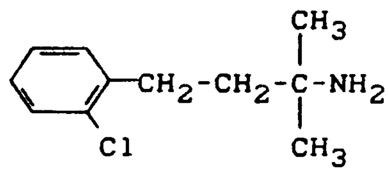

(Verfahren (b))

40,6 g (0,21 Mol) 3-Amino-3,3-dimethyl-1-(2-chlor-phenyl)-1-propin werden in einem Rührautoklaven mit 250 ml Tetrahydrofuran vermischt und mit 10 g Raney-Nickel versetzt. Wasserstoff wird dann bis zu einem Druck von 50 bar eindosiert und das Gemisch unter Rühren allmählich von 25 °C auf 40 °C erwärmt. Der Wasserstoffdruck wird jeweils nach Absinken auf 40 bar immer wieder auf 50 bar eingestellt, bis der Druck konstant bleibt. Anschließend wird filtriert, das Filtrat im Wasserstrahlvakuum eingeengt und das verbleibende Rohprodukt im Ölpumpenvakuum destilliert.

Man erhält 31,8 g (77% der Theorie) 3-Amino-3,3-dimethyl-1-(2-chlor-phenyl)-propan vom Brechungsindex $n_D^{21}$ = 1,4817.

Analog zu den Beispielen 1 bis 3 und entsprechend der allgemeinen Beschreibung des erfindungsgemäßen Herstellungsverfahrens wurden auch die in der nachstehenden Tabelle 1 aufgeführten Verbindungen der Formel (I) hergestellt.

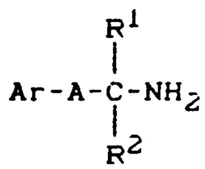

(I)

<u>Tabelle 1:</u>

| Bsp.- Nr. | Ar | A | $R^1$ | $R^2$ | Physikalische Daten |
|---|---|---|---|---|---|
| 4 | 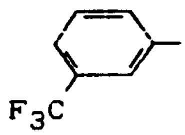 | -C≡C- | $CH_3$ | $CH_3$ | Kp: 72° C-74° C (bei 0,2 mbar) |
| 5 | 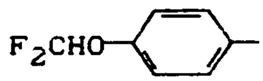 | -C≡C- | $CH_3$ | $CH_3$ | Kp: 88° C-90° C (bei 0,2 mbar) |
| 6 | 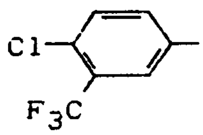 | -C≡C- | $CH_3$ | $CH_3$ | Kp: 84° C-86° C (bei 0,08 mbar) |
| 7 | 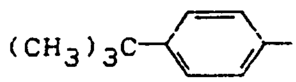 | -C≡C- | $CH_3$ | $CH_3$ | Fp: 57° C-59° C |
| 8 | 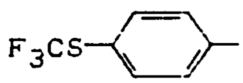 | -C≡C- | $CH_3$ | $CH_3$ | Kp: 72° C (bei 0,05 mbar) |
| 9 | 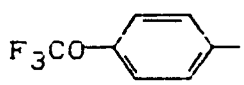 | -C≡C- | $CH_3$ | $CH_3$ | Kp: 75° C-76° C (bei 1 mbar) |
| 10 | 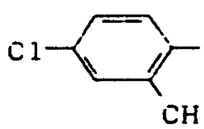 | -C≡C- | $CH_3$ | $CH_3$ | Kp: 99° C-102° C (bei 0,6 mbar) |
| 11 | 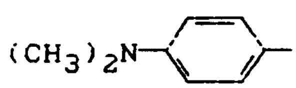 | -C≡C- | $CH_3$ | $CH_3$ | Fp: 79° C-81° C |
| 12 | 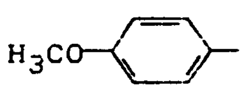 | -C≡C- | $CH_3$ | $CH_3$ | Fp: 44° C-46° C |
| 13 | 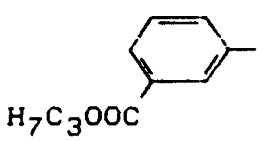 | -C≡C- | $CH_3$ | $CH_3$ | Kp: 155° C (bei 2 mbar) |

Tabelle 1 - Fortsetzung

| Bsp.-Nr. | Ar | A | $R^1$ | $R^2$ | Physikalische Daten |
|---|---|---|---|---|---|
| 14 | 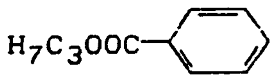 | -C≡C- | $CH_3$ | $CH_3$ | Kp: 149° C (bei 0,5 mbar) |
| 15 | 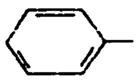 | -C≡C- | $CH_3$ | $CH_3$ | Kp: 73° C (bei 0,3 mbar) |
| 16 | 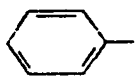 | -C≡C- | $-(CH_2)_5-$ | | Kp: 123° C (bei 0,8 mbar) |
| 17 | 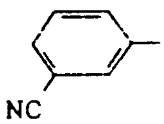 | -C≡C- | $CH_3$ | $CH_3$ | Fp: 63° C |
| 18 | 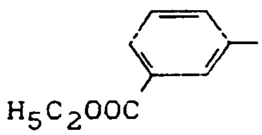 | -C≡C- | $CH_3$ | $CH_3$ | Kp: 128° C (bei 0,2 mbar) |
| 19 | 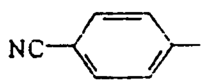 | -C≡C- | $CH_3$ | $CH_3$ | Fp: 84° C |
| 20 | 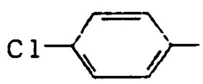 | -C≡C- | $CH_3$ | $CH_3$ | Fp: 32° C |
| 21 | 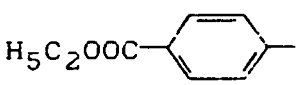 | -C≡C- | $CH_3$ | $CH_3$ | Kp: 145° C (bei 1 mbar) |
| 22 | 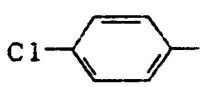 | -C≡C- | $C_2H_5$ | $C_2H_5$ | Kp: 122° C (bei 0,5 mbar) |
| 23 | 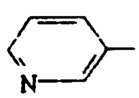 | -C≡C- | $CH_3$ | $CH_3$ | Kp: 75-78° C (bei 0,05 mbar) |

Tabelle 1 - Fortsetzung

| Bsp.-Nr. | Ar | A | $R^1$ | $R^2$ | Physikalische Daten |
|---|---|---|---|---|---|
| 24 | 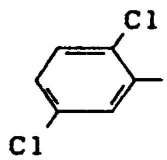 | -C≡C- | $CH_3$ | $CH_3$ | Kp: 135-140° C (bei 0,5 mbar |
| 25 | 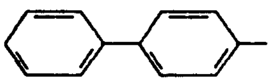 | -C≡C- | $CH_3$ | $CH_3$ | Fp: 78-80° C |
| 26 | 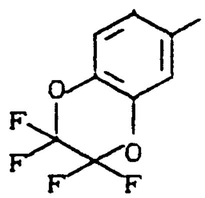 | -C≡C- | $CH_3$ | $CH_3$ | Kp: 88° C (bei 1-2 mbar) Fp:42-44° C |
| 27 | 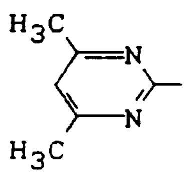 | -C≡C- | $CH_3$ | $CH_3$ | Fp: 63-64° C |
| 28 | 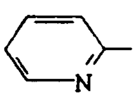 | -C≡C- | $CH_3$ | $CH_3$ | Fp: 70-73° C |

Beispiel 29

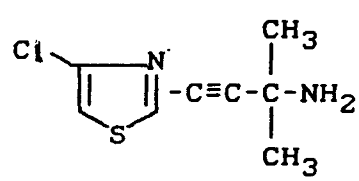

46,2 g (0,30 Mol) 2,4-Dichlor-thiazol und 24,9 g (0,30 Mol) 3-Amino-3, 3-dimethyl-1-propin werden in 300 ml Triethylamin vorgelegt. Nach Zugabe von 2,1 g (0,003 Mol) Palladium (II)-bis(triphenylphosphin)-dichlorid, 2,28 g (0,012 Mol) Kupfer(I)-iodid und 3,2 g (0,012 Mol) Triphenylphosphin wird das Reaktionsgemisch 19 Stunden unter Rückfluß erhitzt. Dann wird filtriert, das Filtrat im Wasserstrahlvakuum eingeengt, der Rückstand in Chloroform aufgenommen und mit verdünnter wäßriger Salzsäure ausgeschüttelt. Die wäßrige Phase wird mit Natronlauge alkalisch gestellt und mehrfach mit Chloroform ausgeschüttelt. Nach Trocknen der organischen Phase über Natriumsulfat wird eingeengt und der Rückstand über Kieselgel chromatographiert (Laufmittel : Methylenchlorid/Methanol (96/4).
Man erhält 15,6 g (26% der Theorie) 3-Amino-3, 3-dimethyl-1-(4-chlor-thiazol-2-yl)-1-propin vom Festpunkt 62-64 ° C.

Beispiel 30

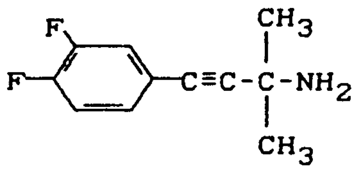

155 g (0,8 Mol) 3,4-Difluor-brombenzol und 75 g (0,9 Mol) 3-Amino-3, 3-dimethyl-1-propin werden in 900 ml Triethylamin vorgelegt. Nach Zugabe von 2,81 g (0,004 Mol) Palladium(II)-bis(triphenylphoshin)-dichlorid, 3,05 g (0,016 Mol) Kupfer(I)-iodid und 8,4 g (0,032 Mol) Triphenylphosphin wird das Reaktionsgemisch 4 Stunden unter Rückfluß erhitzt. Anschließend wird filtriert, das Filtrat im Wasserstrahlvakuum eingeengt und der Rückstand im Ölpumpenvakuum destilliert.

Man erhält 137 g (86% der Theorie) 3-Amino-3,3-dimethyl-1-(3,4-difluor-phenyl)-1-propin vom Siedepunkt 75 °C bei 1,5 mbar.

Analog zu dem Beispiel 30 wurden auch die folgend aufgeführten Verbindungen der Formel (I) hergestellt.

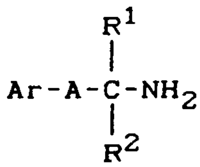 (I)

(Fortsetzung von Tabelle 1)

| Beispiel Nr. | Ar | A | $R^1$ | $R^2$ | Physikalische Daten |
|---|---|---|---|---|---|
| 31 | 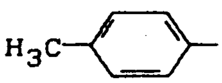 | -C≡C- | $CH_3$ | $CH_3$ | Kp: 102° C (bei 1,5 mbar) |
| 32 | 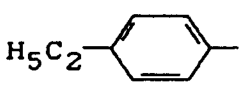 | -C≡C- | $CH_3$ | $CH_3$ | Kp: 82-88° C (bei 0,8 mbar) |
| 33 | 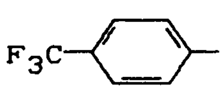 | -C≡C- | $CH_3$ | $CH_3$ | Kp: 79° C (bei 1,5 mbar) Fp.41-42° C |
| 34 | 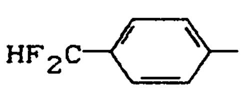 | -C≡C- | $CH_3$ | $CH_3$ | Fp. 36-37° C |
| 35 | 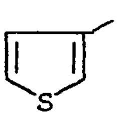 | -C≡C- | $CH_3$ | $CH_3$ | Kp: 77-78° C (bei 0,6 mbar) |

Beispiel zur Verwendung als Zwischenprodukte:

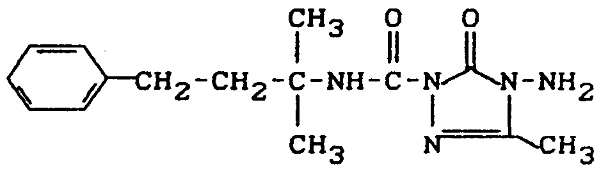

Eine Mischung aus 5,5 g (0,02 Mol) 4-Isopropylidenimino-3-methyl-1-phenoxycarbonyl-1,2,4-triazolin-5-on (bekannt aus EP-A 294666, Beispiel X-1, S. 48), 3,3 g (0,02 Mol) 3-Amino-3,3-dimethyl-1-phenyl-propan und 100 ml Tetrahydrofuran wird 18 Stunden bei 20 °C gerührt und anschließend im Wasserstrahlvakuum eingeengt. Zum verbleibenden Rückstand werden 100 ml Ethanol, 20 ml Wasser und 10 ml konz. Salzsäure gegeben und dieses Gemisch wird 3 Stunden bei 60 °C/200 mbar gerührt und eingeengt. Der hierbei verbleibende Rückstand wird in 100 ml Methylenchlorid aufgenommen, diese Lösung mit 50 ml gesättigter wäßriger Natriumhydrogencarbonat-Lösung extrahiert, anschließend mit Magnesiumsulfat getrocknet und filtriert. Das Filtrat wird im Wasserstrahlvakuum eingeengt, der Rückstand durch Verreiben mit Diethylether zur Kristallisation gebracht und das kristalline Produkt durch Absaugen isoliert.

Man erhält 3,64 g (60% der Theorie) 1-(1,1-Dimethyl-3-phenyl-propyl-amino-carbonyl)-3-methyl-4-amino-1,2,4-triazolin-5-on vom Schmelzpunkt 87 °C.

Diese Verbindung ist als Herstellungsbeispiel (Nr. 132) in EP-A 294666 aufgeführt; sie kann als Herbizid verwendet werden.

**Patentansprüche**

**1.** Verfahren zur Herstellung von (Hetero)Arylalk(en/in)-ylaminen der allgemeinen Formel (I)

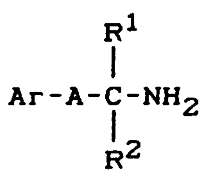

(I)

in welcher

| | |
|---|---|
| A | für Ethan-1,2-diyl (Ethylen, Dimethylen, $-CH_2-CH_2-$) oder für Ethen-1,2-diyl (Ethenylen, Vinylen, $-CH=CH-$) oder für Ethin-1,2-diyl (Ethinylen, $-C\equiv C-$) steht, |
| Ar | für jeweils gegebenenfalls substituiertes Aryl oder Heteroaryl steht, sowie |
| $R^1$ und $R^2$ | gleich oder verschieden sind und einzeln für Wasserstoff oder Alkyl stehen oder zusammen für Alkandiyl (Alkylen) stehen, |

dadurch gekennzeichnet, daß man

(a) für den Fall, daß in der Formel (I) A für Ethin-1,2-diyl steht und Ar, $R^1$ und $R^2$ die oben angegebene Bedeutung haben,

Halogen(hetero)arylverbindungen der allgemeinen Formel (II)

Ar-X (II)

in welcher

| | |
|---|---|
| Ar | die oben angegebene Bedeutung hat und |
| X | für Halogen steht, |

mit Aminoalkinylverbindungen der allgemeinen Formel (III)

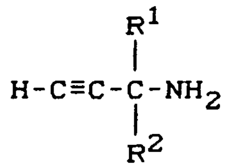

(III)

in welcher

$R^1$ und $R^2$ die oben angegebene Bedeutung haben,

in Gegenwart eines Katalysators, in Gegenwart eines Säureakzeptors, gegebenenfalls in Gegenwart weiterer Reaktionshilfsmittel und gegebenenfalls in Gegenwart eines Verdünnungsmittels bei Temperaturen zwischen 0 ° C und 200 ° C umsetzt,

und gegebenenfalls

(b) für den Fall, daß in der Formel (I) A für Ethan-1,2-diyl oder Ethen-1,2-diyl steht und Ar, $R^1$ und $R^2$ die oben angegebene Bedeutung haben,

die nach dem unter (a) beschriebenen Verfahrensschritt erhaltenen Verbindungen der allgemeinen Formel (Ia)

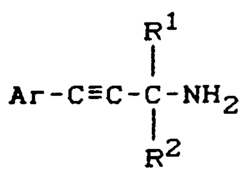

(Ia)

in welcher

Ar, $R^1$ und $R^2$ die oben angegebene Bedeutung , haben

mit einem Hydrierungsmittel, gegebenenfalls in Gegenwart eines Katalysators und gegebenenfalls in Gegenwart eines Verdünnungsmittels, bei Temperaturen zwischen 0 ° C und 150 ° C umsetzt.

**2.** Verfahren zur Herstellung von (Hetero)Aralk(en/in)-ylaminen der allgemeinen Formel (I) gemäß Anspruch 1, in welcher

A für Ethan-1,2-diyl (Ethylen, Dimethylen, $-CH_2-CH_2-$) oder für Ethen-1,2-diyl (Ethenylen, Vinylen, $-CH=CH-$) oder für Ethin-1,2-diyl (Ethinylen, $-C\equiv C-$) steht,

Ar für jeweils gegebenenfalls substituiertes Aryl oder Heteroaryl mit jeweils bis zu 10 Kohlenstoffatomen und gegebenenfalls 1 bis 3 Stickstoffatomen und/oder einem Sauerstoff- oder Schwefelatom als Heteroatom(en) steht, wobei als bevorzugte Substituenten ausgewählt sind: Halogen, Cyano, Nitro, jeweils geradkettiges oder verzweigtes Alkyl, Alkoxy, Alkylthio oder Dialkylamino mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 8 Kohlenstoffatomen in den einzelnen Alkylteilen, Cycloalkyl mit 3 bis 7 Kohlenstoffatomen, zweifach verknüpftes Alkandiyl mit 3 bis 5 Kohlenstoffatomen, gegebenenfalls einfach bis vierfach durch Fluor und/- oder Chlor substituiertes Alkylendioxy mit 1 oder 2 Kohlenstoffatomen, jeweils gegebenenfalls im Arylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy, Aryloxyalkyl oder Aralkyloxy mit jeweils 6 bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen

substituiertes Heteroarylalkyl oder Heteroaryl mit jeweils 2 bis 9 Kohlenstoffatomen und 1 bis 4 gleichen oder verschiedenen Heteroatomen - insbesondere Stickstoff, Sauerstoff und/oder Schwefel - im Heteroarylteil und gegebenenfalls 1 bis 4 Kohlenstoffatomen im geradkettigen oder verzweigten Alkylteil; sowie

$R^1$ und $R^2$ gleich oder verschieden sind und einzeln für Wasserstoff oder für geradkettiges oder verzweigtes Alkyl mit 1 bis 6 Kohlenstoffatomen stehen oder zusammen für Alkandiyl (Alkylen) mit 2 bis 6 Kohlenstoffatomen stehen.

**3.** Verfahren zur Herstellung von (Hetero)Aralk(en/in)-ylaminen der allgemeinen Formel (I) gemäß Anspruch 1, in welcher

A für Ethan-1,2-diyl (Ethylen, Dimethylen, $-CH_2-CH_2-$) oder für Ethen-1,2-diyl (Ethenylen, Vinylen, $-CH=CH-$) oder für Ethin-1,2-diyl (Ethinylen, $-C\equiv C-$) steht,

Ar für jeweils gegebenenfalls einfach bis dreifach substituiertes Phenyl, Naphthyl, Pyridyl, Pyrimidyl, Triazinyl, Furyl, (Iso)-Oxazolyl, Oxadiazolyl, Thienyl, (Iso)Thiazolyl oder Thiadiazolyl steht, wobei als besonders bevorzugte Substituenten ausgewählt sind: Fluor, Chlor, Brom, Cyano, Methyl, Ethyl, n-oder i-Propyl, n-, i-, s- oder t-Butyl, Methoxy, Ethoxy, n- oder i-Propoxy, Methylthio, Ethylthio, n- oder i-Propylthio, Dimethylamino, Difluormethyl, Trifluormethyl, Difluormethoxy, Trifluormethoxy, Difluormethylthio, Trifluormethylthio, Methoxycarbonyl, Ethoxycarbonyl, n- oder i-Propoxycarbonyl, Methoximinomethyl, Ethoximinomethyl, Methoximinoethyl, Ethoximinoethyl, Cyclohexyl oder jeweils gegebenenfalls im Phenylteil ein- bis dreifach, gleich oder verschieden durch Fluor, Chlor, Brom, Methyl, Ethyl, Methoxy, Ethoxy, Methylthio, Trifluormethyl, Difluormethoxy, Trifluormethoxy und/oder Trifluormethylthio substituiertes Phenyl, Benzyl, Phenoxy, Phenoxymethyl oder Benzyloxy,

$R^1$ für Wasserstoff, Methyl, Ethyl, n- oder i-Propyl steht und

$R^2$ für Methyl, Ethyl, n- oder i-Propyl, n- oder i-Butyl steht, oder zusammen mit $R^1$ für Ethan-1,2-diyl (Dimethylen), Propan-1,3-diyl (Trimethylen), Butan-1,4-diyl (Tetramethylen) oder Pentan-1,5-diyl (Pentamethylen) steht.

**4.** Verfahren zur Herstellung von (Hetero)Arylalk(en/in)-ylaminen gemäß Ansprüche 1 bis 3, dadurch gekennzeichnet, daß das erfindungsgemäße Verfahren (b) in Gegenwart eines Katalysators durchgeführt wird.

**5.** Verfahren zur Herstellung von (Hetero)Arylalk(en/in)-ylaminen gemäß der Ansprüche 1 bis 3, dadurch gekennzeichnet, daß das erfindungsgemäße Verfahren in Gegenwart weiterer Reaktionshilfsmittel durchgeführt wird.

**6.** Verfahren zur Herstellung von (Hetero)Arylalk(en/in)-ylaminen gemäß Ansprüche 1 bis 3, dadurch gekennzeichnet, daß das erfindungsgemäße Verfahren in Gegenwart eines Verdünnungsmittels durchgeführt wird.

**7.** Verfahren gemäß Ansprüche 1 bis 3, dadurch gekennzeichnet, daß man das erfindungsgemäße Verfahren (b) bei Normaldruck oder erhöhtem Druck bis etwa 200 bar durchführt.

**8.** (Hetero)Aralkinylamine der Formel (Ia)

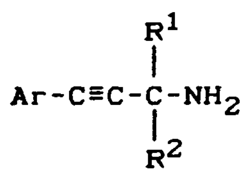

(Ia)

in welcher

Ar für jeweils gegebenenfalls substituiertes Aryl oder Heteroaryl steht und

$R^1$ und $R^2$ gleich oder verschieden sind und einzeln für Wasserstoff oder Alkyl stehen oder zusammen für Alkandiyl (Alkylen) stehen,

mit Ausnahme von 3-Phenyl-2-propinylamin und 1-Methyl-3-phenyl-2-propinylamin sowie N'-[3-(3-Amino-3-methyl-1-butinyl)-phenyl]-N-methoxy-N-methylharnstoff.

**9.** (Hetero)Aralkinylamine der Formel (Ia) gemäß Anspruch 8, in welcher

Ar für jeweils gegebenenfalls substituiertes Aryl oder Heteroaryl mit jeweils bis zu 10 Kohlenstoffatomen und gegebenenfalls 1 bis 3 Stickstoffatomen und/oder einem Sauerstoff- oder Schwefelatom als Heteroatom(en) steht, wobei als bevorzugte Substituenten ausgewählt sind: Halogen, Cyano, Nitro, jeweils geradkettiges oder verzweigtes Alkyl, Alkoxy, Alkylthio oder Dialkylamino mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 8 Kohlenstoffatomen in den einzelnen Alkylteilen, Cycloalkyl mit 3 bis 7 Kohlenstoffatomen, zweifach verknüpftes Alkandiyl mit 3 bis 5 Kohlenstoffatomen, gegebenenfalls einfach bis vierfach durch Fluor und/oder Chlor substituiertes Alkylendioxy mit 1 oder 2 Kohlenstoffatomen, jeweils gegebenenfalls im Arylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Aryl, Aralkyl, Aryloxy, Aryloxyalkyl oder Aralkyloxy mit jeweils 6 bis 10 Kohlenstoffatomen im Arylteil und gegebenenfalls 1 bis 4 Kohlenstoffatomen im geradkettigen oder verzweigten Alkylteil oder jeweils gegebenenfalls im Heteroarylteil einfach oder mehrfach, gleich oder verschieden durch Halogen, Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen substituiertes Heteroarylalkyl oder Heteroaryl mit jeweils 2 bis 9 Kohlenstoffatomen und 1 bis 4 gleichen oder verschiedenen Heteroatomen - insbesondere Stickstoff, Sauerstoff und/oder Schwefel - im Heteroarylteil und gegebenenfalls 1 bis 4 Kohlenstoffatomen im geradkettigen oder verzweigten Alkylteil; sowie

$R^1$ und $R^2$ gleich oder verschieden sind und einzeln für Wasserstoff oder für geradkettiges oder verzweigtes Alkyl mit 1 bis 6 Kohlenstoffatomen stehen oder zusammen für Alkandiyl (Alkylen) mit 2 bis 6 Kohlenstoffatomen stehen,

mit der Maßgabe, daß 3-Phenyl-2-propinylamin und 1-Methyl-3-phenyl-2-propinylamin ausgenommen sind.

**10.** (Hetero)Aralkinylamine der Formel (Ia) gemäß Anspruch 8, in welcher

Ar für jeweils gegebenenfalls einfach bis dreifach substituiertes Phenyl, Naphthyl, Pyridyl, Pyrimidyl, Triazinyl, Furyl, (Iso)-Oxazolyl, Oxadiazolyl, Thienyl, (Iso)Thiazolyl oder Thiadiazolyl steht, wobei als besonders bevorzugte Substituenten ausgewählt sind: Fluor, Chlor, Brom, Cyano, Methyl, Ethyl, n-oder i-Propyl, n-, i-, s- oder t-Butyl, Methoxy, Ethoxy, n- oder i-Propoxy, Methylthio, Ethylthio, n- oder i-Propylthio, Dimethylamino, Difluormethyl, Trifluormethyl, Difluormethoxy, Trifluormethoxy, Difluormethylthio, Trifluormethylthio, Methoxycarbonyl, Ethoxycarbonyl, n- oder i-Propoxycarbonyl, Methoximinomethyl, Ethoximinomethyl, Methoximinoethyl, Ethoximinoethyl, Cyclohexyl oder jeweils gegebenenfalls im Phenylteil ein- bis dreifach, gleich oder verschieden durch Fluor, Chlor, Brom, Methyl, Ethyl, Methoxy, Ethoxy, Methylthio, Trifluormethyl, Difluormethoxy, Trifluormethoxy und/oder Trifluormethylthio substituiertes Phenyl, Benzyl, Phenoxy, Phenoxymethyl oder Benzyloxy,

$R^1$ für Wasserstoff, Methyl, Ethyl, n- oder i-Propyl steht und

$R^2$ für Methyl, Ethyl, n- oder i-Propyl, n- oder i-Butyl steht, oder zusammen mit $R^1$ für Ethan-1,2-diyl (Dimethylen), Propan-1,3-diyl (Trimethylen), Butan-1,4-diyl (Tetramethylen) oder Pentan-1,5-diyl (Pentamethylen) steht,

mit der Maßgabe, daß 3-Phenyl-2-propinylamin und 1-Methyl-3-phenyl-2-propinylamin ausgenommen sind.

Europäisches Patentamt

# EUROPÄISCHER RECHERCHENBERICHT

Nummer der Anmeldung

EP 92 10 0482
Seite 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | JOURNAL OF ORGANIC CHEMISTRY.<br>Bd. 54, Nr. 14, 1989, WASHINGTON DC US<br>Seiten 3420 - 22;<br>Hobbs, Frank W., Jr.: 'Palladium-catalyzed synthesis of alkynylamino nucleosides. A universal linker for nucleic acids'<br>* das ganze Dokument * | 1-10 | C07C211/28<br>C07C211/29<br>C07C209/68 |
| X | J. ORG. CHEM., 51(12), 2191-202<br>1986,<br>Trybulski, E. J.; Fryer, R. I.; Reeder, E.; Vitone, S.; Todaro, L.: '2-Benzazepines. 9. Synthesis and chemistry of 3H-2-benzazepine and pyrimido[4,5-d][2]benzazepine derivatives'<br>* Seite 2191: Scheme I * | 1-10 | |
| D,X | EP-A-0 030 922 (CIBA-GEIGY AG.)<br>* Seite 5 *<br>* Seite 19; Beispiele 2.70 * | 1-7 | |
| X | WO-A-8 810 264 (HOWARD FLOREY INSTITUTE OF EXPERIMENTAL PHYSIOLOGY AND MEDICINE)<br>* Anspruch 14 *<br>* Seite 25, Zeile 24 - Seite 26, Zeile 15 *<br>* Seite 33 * | 1-10 | **RECHERCHIERTE SACHGEBIETE (Int. Cl.5 )**<br><br>C07C |
| X | EP-A-0 251 786 (E.I. DU PONT DE NEMOURS AND CO.)<br>* Beispiel 13 * | 1-10 | |
| X | J. ORG. CHEM., 41(24), 3813-19<br>1976,<br>Seiten 3813 - 3919;<br>Klemm, LeRoy H.; Hwang, Yoon Ni; McGuire, Thomas M.: 'Intramolecular Diels-Alder reactions. 12. Competitive [4 + 2] and [2 + 2] cycloadditions of N-(phenylpropargyl)-cis-cinnamamide'<br>* Seite 3818; Beispiel 10 * | 8-10 | |
| X | US-A-3 332 988 (ROBERT PAUL MULL)<br>* Spalte 17, Zeile 16 - Zeile 60 * | 8-10 | |

Der vorliegende Recherchenbericht wurde für alle Patentansprüche erstellt

| Recherchenort | Abschlußdatum der Recherche | Prüfer |
|---|---|---|
| DEN HAAG | 15 MAI 1992 | PAUWELS G.R.A. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 92 10 0482
Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| | --- | | |
| X | US-A-4 743 617 (THOMAS M. BARGAR)<br>* Ansprüche *<br>--- | 8-10 | |
| X | EP-A-0 252 683 (E.I. DU PONT DE NEMOURS AND CO.)<br>* Beispiele 5-8,11 *<br>--- | 8-10 | |
| X | TETRAHEDRON LETT., 23(43), 4517-20<br>1982,<br>Catellani, Marta; Chiusoli, Gian Paolo: 'One-pot palladium-catalyzed synthesis of 2,3-disubstituted bicyclo[2.2.1]heptanes and bicyclo[2.2.1]hept-5-enes'<br>* Seite 4519, Zeile 1 - Zeile 4 *<br>--- | 1-7 | |
| X | AGENTS ACTIONS, 17(2), 138-44<br>1985,<br>Banning, Jon W.; Griffith, Robert K.; Dipietro, Richard A.: 'Histamine receptor activation by unsaturated (allyl and propargyl) homologs of histamine'<br>* Seite 138: Figure I *<br>--- | 8-10 | RECHERCHIERTE SACHGEBIETE (Int. Cl.5 ) |
| A | J. MED. CHEM., 13(6), 1249-50<br>1970,<br>Simon, David Z.; Salvador, Romano L.; Champagne, Gaston: 'Acetylenics. 1. Aromatic amines containing the acetylenic triple bond'<br>* das ganze Dokument *<br>----- | 8-10 | |

Der vorliegende Recherchenbericht wurde für alle Patentansprüche erstellt

| Recherchenort | Abschlußdatum der Recherche | Prüfer |
|---|---|---|
| DEN HAAG | 15 MAI 1992 | PAUWELS G.R.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument